# EUROPEAN PATENT SPECIFICATION

(11) **EP 2 321 406 B1**
(45) Date of publication and mention of the grant of the patent: **10.12.2014**
(21) Application number: 09804740.0
(22) Date of filing: 05.08.2009
(51) Int. Cl.: C12N 5/079, G01N 33/48, C12Q 1/68

(54) **METHOD FOR SELECTING SECONDARY NEUROSPHERE DERIVED FROM INDUCED PLURIPOTENT STEM CELL**
VERFAHREN ZUR AUSWAHL EINER SEKUNDÄREN NEUROSPHÄRE AUS EINER INDUZIERTEN PLURIPOTENTEN STAMMZELLE
PROCÉDÉ DE SÉLECTION D'UNE NEUROSPHÈRE SECONDAIRE ISSUE D'UNE CELLULE SOUCHE PLURIPOTENTE INDUITE

(30) Priority: 05.08.2008 US 86369 P
(43) Date of publication of application: 18.05.2011
(73) Proprietor: KEIO UNIVERSITY, Tokyo 108-8345 (JP); KYOTO UNIVERSITY, Kyoto 606-8501 (JP)
(72) Inventor: OKANO, Hideyuki, Tokyo 160-8582 (JP); TSUJI, Osahiko, Tokyo 160-8582 (JP); NAKAMURA, Masaya, Tokyo 160-8582 (JP); YAMANAKA, Shinya, Kyoto-shi, Kyoto 606-8501 (JP); MIURA, Kyoko, Kyoto-shi, Kyoto 606-8501 (JP)
(74) Representative: Creek, Isobel Clare
(86) International application number: PCT/JP2009/003755
(87) International publication number: WO 2010/016253

(56) References cited:
- WO-A1-03/095629
- WO-A2-2007/047581
- CHUNG S ET AL: "Genetic selection of sox1GFP-expressing neural precursors removes residual tumorigenic pluripotent stem cells and attenuates tumor formation after transplantation", JOURNAL OF NEUROCHEMISTRY, WILEY INTERSCIENCE, NEW YORK, NY, US, vol. 97, no. 5, 1 June 2006 (2006-06-01), pages 1467-1480, XP008143938, ISSN: 0022-3042, DOI: 10.1111/J.1471-4159.2006.03841.X [retrieved on 2006-04-28]
- HITOSHI FUKUDA ET AL: "Fluorescence-Activated Cell Sorting-Based Purification of Embryonic Stem Cell-Derived Neural Precursors Averts Tumor Formation after Transplantation", STEM CELLS, vol. 24, no. 3, 1 March 2006 (2006-03-01), pages 763-771, XP55016441, ISSN: 1066-5099, DOI: 10.1634/stemcells.2005-0137
- RAQUEL MARTÍN-IBÁÑEZ ET AL: "Interplay of leukemia inhibitory factor and retinoic acid on neural differentiation of mouse embryonic stem cells", JOURNAL OF NEUROSCIENCE RESEARCH, vol. 85, no. 12, 1 September 2007 (2007-09-01), pages 2686-2701, XP55022109, ISSN: 0360-4012, DOI: 10.1002/jnr.21228
- KEISUKE OKITA ET AL: "Generation of germline-competent induced pluripotent stem cells", NATURE: INTERNATIONAL WEEKLY JOURNAL OF SCIENCE, NATURE PUBLISHING GROUP, UNITED KINGDOM, vol. 448, 1 July 2007 (2007-07-01), pages 313-317, XP008137105, ISSN: 0028-0836
- PIESTUN D ET AL: "Nanog transforms NIH3T3 cells and targets cell-type restricted genes", BIOCHEMICAL AND BIOPHYSICAL RESEARCH COMMUNICATIONS, ACADEMIC PRESS INC. ORLANDO, FL, US, vol. 343, no. 1, 28 April 2006 (2006-04-28), pages 279-285, XP024923566, ISSN: 0006-291X, DOI: 10.1016/J.BBRC.2006.02.152 [retrieved on 2006-04-28]
- MIURA, K ET AL.: 'Variation in the safety of induced pluripotent stem cell lines.' NAT BIOTECHNOL. vol. 27, no. 8, August 2009, pages 743 - 745, XP008143890
- EIGES, R ET AL.: 'Establishment of human embryonic stem cell-transfected clones carrying a marker for undifferentiated cells.' CURR BIOL. vol. 11, no. 7, 03 April 2001, pages 514 - 518, XP001155602
- OKITA,K ET AL.: 'Generation of germline-competent induced pluripotent stem cells.' NATURE vol. 448, no. 7151, 19 July 2007, pages 313 - 317, XP008137105
- OKANO,H. ET AL.: 'A study of imaging and therapy in <?PIHORS-JCR-BEGINcitations_suite ?> Amyotrophic Lateral Sclerosis (ALS) Regeneration of the central nervous system: using embryonic stem cells and neural stem cells.' A STUDY OF IMAGING AND THERAPY IN AMYOTROPHIC LATERAL SCLEROSIS(ALS) ANNUAL REPORT 2007: SUMMARY OF THE RESEARCH [IN JAPANESE]. March 2008, pages 17 - 19, XP008144460
- OKADA, Y ET AL.: 'Spatiotemporal recapitulation of central nervous system development by murine embryonic stem cell-derived neural stem/progenitor cells.' STEM CELLS. vol. 26, no. 12, December 2008, pages 3086 - 3098, XP008143940
- TANABE,K ET AL.: 'Ahistrical overview and future prospects of the iPS cells.' REGENERATIVE MEDICINE [IN JAPANESE] vol. 7, no. 2, 01 May 2008, pages 83 - 89, XP008144400
- CHUNG,S ET AL.: 'Genetic selection of soxlGFP-expressing neural precursors removes residual tumorigenic pluripotent stem cells and attenuates tumor formation after transplantation.' J NEUROCHEM. vol. 97, no. 5, June 2006, pages 1467 - 1480, XP008143938

## Description

### Technical Filed

The present invention relates to methods for selecting a secondary neurosphere derived from a differentiated cell-derived pluripotent stem cell.

### Background Art

Recently it has become possible to obtain cells having pluripotency similar to embryonic stem cells (hereinafter referred to as ES cells) by selecting cells expressing Fbx15 gene from somatic cells such as fibroblast to which Oct3/4 gene, Sox2 gene, Klf4 gene and c-myc gene have been introduced and expressed (Takahashi K, Yamanaka S. (2006). "Induction of pluripotent stem cells from mouse embryonic and adult fibroblast cultures by defined factors." Cell 126: 663-676). It is considered that if the pluripotent stem cells derived from the somatic cells thus obtained are used in regenerative medicine, patients' own cells can be transplanted, thereby minimizing problems of rejections compared with the cases where ES cells are used.

While the induced pluripotent stem cells (also referred to as iPS cells) produced by using Fbx15 gene as a marker were closely similar to embryonic stem cells in characteristics such as cell morphology, proliferation ability, and differentiation ability, they were different from ES cells in terms of some characteristics such as gene expression and DNA methylation patterns. Next by selecting cells using the expression of the Nanog gene as a marker, lines of iPS cells having pluripotency more similar to ES cells were established (Okita K, Ichisaka T, Yamanaka S. (2007). "Generation of germline-competent induced pluripotent stem cells." Nature 448: 313-317).

Later, iPS cells were generated by using changes in cell morphology as a marker, instead of the expression of Fbx15 gene or Nanog gene Meissner A, Wernig M, Jaenisch R. (2007). "Direct reprogramming of genetically unmodified fibroblasts into pluripotent stem cells." Nat Biotechnol 25: 1177-1181). Other iPS cells were further generated by using N-myc instead of c-myc (Blelloch R, Venere M, Yen J, Ramalho-Santos M. (2007). "Generation of induced pluripotent stem cells in the absence of drug selection." Cell Stem Cell 1: 245-247). In addition, iPS cells were produced from hepatocytes and gastric epithelial cells instead of fibroblasts (Aoi T, Nakagawa M, Ichisaka T, Okita K, Takahashi K, Chiba T, Yamanaka S. (2008). "Generation of pluripotent stem cells from adult mouse liver and stomach cells." Science 321: 699-702).

Meanwhile, there has been a growing body of studies on iPS cells using human cells. Human iPS cells were generated by introducing Oct3/4 gene, Sox2 gene, Nanog gene, and lin28 gene into fibroblasts (Yu J, Vodyanik MA, Smuga-Otto K, Antosiewicz-Bourget J, Frane JL, Tian S, Nie J, Jonsdottir GA, Ruotti V, Stewart R, Slukvin II, Thomson JA. (2007). "Induced pluripotent stem cell lines derived from human somatic cells." Science 318: 1917-1920). Human iPS cells were also produced by introducing Oct3/4 gene, Sox2 gene, Klf4 gene, and c-myc gene, which are the same combination of genes as used for establishment of mouse iPS cells, into human fibroblasts or fibroblast-like synoviocytes (Takahashi K, Tanabe K, Ohnuki M, Narita M, Ichisaka T, Tomoda K, Yamanaka S. (2007). "Induction of pluripotent stem cells from adult human fibroblasts by defined factors." Cell 131: 861-872).

Since the iPS cells can be produced using cells derived from a patient to be treated, artificial organs which are free from rejection are expected to be produced by using the iPS cells in the field of regenerative medicine. Cellular behaviors in vivo of the iPS cells, however, suggest that the iPS cells are not the cells having completely the same characteristics as the ES cells. For example, when chimeric mice were generated using iPS cells, tumor formation was observed in about 20% of the mice, a value much higher than that obtained in similar experiments using ES cells.

To alleviate this problem of high risk of tumor formation, iPS cells were produced in human and mice by using Oct3/4, Sox2 and Klf4 without c-myc, thereby reducing the risk of tumor formation in chimeric mice (Nakagawa M, Koyanagi M, Tanabe K, Takahashi K, Ichisaka T, Aoi T, Okita K, Mochiduki Y, Takizawa N, Yamanaka S. (2008). "Generation of induced pluripotent stem cells without Myc from mouse and human fibroblasts". Nat Biotechnol 26: 101-106; Wernig M, Meissner A, Cassady JP, Jaenisch R. (2008). "c-Myc is dispensable for direct reprogramming of mouse fibroblasts". Cell Stem Cell 2: 10-12). However, efficiency of the establishment of iPS cells by using only the three genes are lower than by using the four genes (Nakagawa M, Koyanagi M, Tanabe K, Takahashi K, Ichisaka T, Aoi T, Okita K, Mochiduki Y, Takizawa N, Yamanaka S. (2008). "Generation of induced pluripotent stem cells without Myc from mouse and human fibroblasts". Nat Biotechnol 26: 101-106; Wernig M, Meissner A, Cassady JP, Jaenisch R. (2008). "c-Myc is dispensable for direct reprogramming of mouse fibroblasts". Cell Stem Cell 2: 10-12).
Chung et al, Journal of Neurochemistry, 97(5):1467-1480 (2006) and Fukuda et al, Stem Cells, 24(3):763-771 (2006) each disclose a method for selecting in vitro induced neurospheres from ES cells having a low risk of tumour formation by examining the activity of the Sox-1 gene promoter and selecting those in which the promoter is active.
Raquel Martín-Ibáñez et al, Journal of Neuroscience Research, 85(12):2686-2701 (2007) discloses a method for controlled differentiation of ES cells by exposure to retinoic acid in the absence of LIF and consequent observation of increased neural marker expression and decreased Sox-1 expression.
WO 06/095629 discloses a method of producing neural progenitor cells and neuronal cells from a pluripotent cell population by culturing the pluripotent cells under conditions that promote neuronal differentiation.

### Summary of Invention

### Technical Problem

Accordingly, the present invention is intended to provide methods for efficiently identifying and selecting an induced pluripotent stem cell-derived secondary neurosphere having low or no risk of tumor formation after transplantation in vivo.

### Solution to Problem

In one embodiment of the present invention, a method for selecting a secondary neurosphere having low or no risk of tumor formation after transplantation among secondary neurospheres derived from an induced pluripotent stem cell includes steps of examining promoter activity of Nanog gene in each of the secondary neurospheres and selecting a secondary neurosphere in which the promoter activity of Nanog gene is repressed.

In another embodiment of the present invention, a method for preparing a secondary neurosphere having low or no risk of tumor formation after transplantation includes steps of:
(i)preparing secondary neurospheres using an induced pluripotent stem cell,
(ii)examining promoter activity of Nanog gene in each of the secondary neurospheres, and
(iii) selecting and isolating a secondary neurosphere in which the promoter activity of Nanog gene is repressed. The secondary neurosphere having low or no risk of tumor formation after transplantation can be selected by anyone of the selection methods described above.

In any one of the above methods, the secondary neurospheres may have a marker gene whose expression is regulated by a promoter of Nanog gene, and expression of the marker gene may be examined in the selection of the secondary neurosphere. The marker gene encodes preferably a fluorescent protein, a luminescent protein, or an enzyme. Expression of endogenous Nanog gene may be examined instead of the marker expression.

In any one of the above methods, an activation level of the promoter of Nanog gene may be measured in each cell belonging to each of the secondary neurospheres. Ratio of the cells in which the promoter of Nanog gene is activated may be calculated for each clone of a secondary neurosphere, and a clone whose ratio of cells in which the promoter of Nanog gene is activated is 0.01% or less is preferably selected. Alternatively, an activation level of the promoter of Nanog gene may be measured as a whole in each of the secondary neurospheres

In another embodiment not forming part of the presently claimed invention, a marker for selecting a secondary neurosphere having low or no risk of tumor formation after transplantation among secondary neurospheres derived from an induced pluripotent stem cell is a product expressed by a promoter of Nanog gene. The product may be selected from the group consisting of Nanog protein, a fluorescent protein, a luminescent protein, an enzyme, and a transcription product encoding Nanog protein, a fluorescent protein, a luminescent protein, or an enzyme.

In another embodiment not forming part of the presently claimed invention, a kit for selecting a secondary neurosphere having low or no risk of tumor formation after transplantation among neurospheres derived from an induced pluripotent stem cell includes a reagent for detecting promoter activity of Nanog gene. The reagent may be used for detecting a marker representing the promoter activity of Nanog gene, wherein the marker is selected from the group consisting of Nanog protein, a fluorescent protein, a luminescent protein, an enzyme, and a transcription product encoding Nanog protein, a fluorescent protein, a luminescent protein, or an enzyme.

In another embodiment not forming part of the presently claimed invention, a clone of a secondary neurosphere derived from an induced pluripotent stem cell is provided; in the clone, ratio of cells in which the promoter of Nanog gene is activated may be 0.01 % or less.

In another embodiment not forming part of the presently claimed invention, a pharmaceutical composition containing a secondary neurosphere having low or no risk of tumor formation after transplantation is provided, the neurosphere being derived from an induced pluripotent stem cell, the secondary neurosphere being derived from either one of the above-mentioned clones. The pharmaceutical composition may be a therapeutic agent for nervous system disease, especially nerve injury.

In another embodiment not forming part of the presently claimed invention, a method for treating a nerve injury includes steps of:
(i)selecting, among secondary neurospheres derived from an induced pluripotent stem cell, a secondary neurosphere in which the promoter activity of Nanog gene is repressed, and
(ii)administering the selected secondary neurosphere to a patient suffering from the nerve injury. The selected secondary neurosphere is preferably transplanted to a site of the nerve injury.

It should be noted that the differentiated cell-derived pluripotent stem cell is preferably a cell obtained by forced expression of Oct3/4 gene family, Sox2 gene family and Klf4 gene family in a differentiated cell. The induced pluripotent stem cell is preferably a cell selected by examining the expression of Nanog gene as a marker. The differentiated cell may be a fibroblast.

== CROSS REFERENCE TO RELATED APPLICATIONS == This application claims the benefit of priority to US provisional application US 61/086,369, filed on August 5, 2008.

### Brief Description of Drawings

[fig.1]FIG. 1 is a graph showing time-dependent changes in cell viability in 38C2-Nanog-iPS-SNS-transplantated mice in an example of the present invention.
[fig.2]FIG. 2 shows photographs indicating the result of a histological analysis (hematoxylin and eosin staining) of a transplanted mouse in an example of the present invention.
[fig.3]FIG. 3 shows photographs indicating the result of a histological analysis (DAB staining using HRP-conjugated anti-RFP antibody) of the transplanted mice in an example of the present invention.
[fig.4]FIG. 4 shows photographs indicating the result of a histological analysis (staining with antibodies against cell-type specific markers) of a transplanted mouse in an example of the present invention. As the antibodies against the markers, anti-Hu antibody for detecting neuronal cells (A), anti-GFAP antibody for detecting astrocytes (B), and anti-GST-pi antibody for detecting oligodendrocytes (C) were used.
[fig.5]FIG. 5 shows photographs and a graph indicating the result of a histological analysis (staining with anti-serotonin receptor antibody) of a transplanted mouse in an example of the present invention.
[fig.6]FIG. 6 shows a graph indicating the result of motor function analysis of a transplanted mouse using the BBB scores in an example of the present invention.

### Description of Embodiments

Embodiments of the present invention accomplished based on the above-described findings are hereinafter described in detail by giving examples. Where there is no particular explanations in embodiments or examples, methods described in standard sets of protocols such as J. Sambrook, E. F. Fritsch & T. Maniatis (Ed.), Molecular cloning, a laboratory manual (3rd edition), Cold Spring Harbor Press, Cold Spring Harbor, New York (2001); F. M. Ausubel, R. Brent, R. E. Kingston, D. D. Moore, J.G. Seidman, J. A. Smith, K. Struhl (Ed.), Current Protocols in Molecular Biology, John Wiley & Sons Ltd., or their modified/changed methods are used. When using a commercial reagent kit or a measuring apparatus, unless otherwise explained, protocols attached to them are used.

The object, characteristics, advantages of the present invention as well as the idea thereof are apparent to those skilled in the art from the descriptions given herein, and the present invention can be easily worked by those skilled in the art based on the descriptions given herein. It is to be understood that the embodiments and specific examples of the invention described herein below are to be taken as preferred examples of the present invention. These descriptions are only for illustrative and explanatory purposes and are not intended to limit the invention to these embodiments or examples. It is further apparent to those skilled in the art that various changes and modifications may be made based on the descriptions given herein within the intent and scope of the present invention disclosed herein.

### == Induced pluripotent stem cells ==

An induced pluripotent stem cell refers to a cell having pluripotency and self-reproducing ability, which has been artificially induced by reprogramming a cell other than (a) germline cells (such as egg cells, sperm cells and their precursor cells such as oogonia and spermatogonia) or (b) undifferentiated cells derived from embryos at early stages of development (such as embryonic stem cells). The differentiated cell may be derived from an embryo, a fetus, or an adult, and may originate from any animal species, such as mice or human but is not derived from a human embryo. The characteristics of the differentiated cell are not particularly limited as long as the cell has at least partly lost intrinsic totipotency of a fertilized cell. Examples of the differentiated cell include fibroblasts, epithelial cells, hepatocytes, etc. It should be noted that the induced pluripotent stem cell includes the iPS cells produced with the Yamanaka four factors (Takahashi K, Yamanaka S. (2006). "Induction of pluripotent stem cells from mouse embryonic and adult fibroblast cultures by defined factors." Cell 126: 663-676; Takahashi K, Tanabe K, Ohnuki M, Narita M, Ichisaka T, Tomoda K,Yamanaka S. (2007). "Induction of pluripotent stem cells fromadult human fibroblasts by defined factors." Cell 131: 861-872).

The method for the reprogramming is not particularly limited, but it is preferred that cells are induced by introducing nuclear reprogramming factors such that the cells possess pluripotency and self-reproduction ability. For example, the reprogramming method disclosed in WO2005/080598 or WO2007/069666 can be used.

The nuclear reprogramming factor is not particularly limited, but preferred is a combination of products of the genes specifically expressing in ES cells or the genes involved in the self renewal of ES cell. For example, these genes are selected from each one member of the Oct gene family, Klf gene family, and Sox gene family. In view of efficiency of establishment of the induced pluripotent stem cells, more preferred is a combination further containing a gene product of the myc gene family. The genes belonging to the Oct gene family include Oct3/4, Oct1A, Oct6, etc.; the genes belonging to the Klf gene family include Klt1, Klf2, Klf4, Klf5, etc.; the genes belonging to the Sox gene family include Sox1, Sox2, Sox3, Sox7, Sox15, Sox17, Sox18, etc.; and the genes belonging to the myc gene family include c-myc, N-myc, L-myc, etc. In some cases, the gene product of the myc gene family may be substituted with a cytokine or a chemical compound, and examples of the cytokine include SCF, bFGF, etc.

Examples of the nuclear reprogramming factor other than the above-described combination include a combination containing Nanog gene and lin-28 gene in addition to a gene from the Oct gene family and a gene from the Sox gene family. It should be noted that when introducing the factor, another type of gene product may be introduced in addition to the genes in the above-described combinations. Examples of the gene product include an immortalization-inducing factor etc.

Since all of the above-mentioned genes are highly conserved among the vertebrates, a gene referred herein includes its homologues unless the name of a particular animal is indicated. Moreover, mutated genes including polymorphic genes are also encompassed as long as they have a function comparable to that of the wild-type gene product.

### == Methods for preparing induced pluripotent stem cells ==

When preparing induced pluripotent stem cells by using nuclear reprogramming factor, in the case that the nuclear reprogramming factor is a protein functioning in a cell it is preferred that a gene encoding the protein is introduced into target differentiated cells such as somatic cells and intracellularly expressed by using an expression vector, (the gene transduction method). The expression vector to be used is not particularly limited, but preferred is a viral vector, and particularly preferred is a retroviral vector, a lentiviral vector, adenoviral vector or Sendai viral vector. Also, the nuclear reprogramming factor may be bound to a peptide called protein transduction domain (PTD) and introduced into cells by adding the fusion protein to a culture medium (the protein transduction method). In the case that the factor functions extracellularly, it may be added to a culture medium of differentiated cells during preparating the induced pluripotent stem cells. If the nuclear reprogramming factor is expressed in the differentiated cells, such as neural stem cells, to be reprogrammed, it is not necessary to introduce it from the outside.

Then, cells expressing an undifferentiation marker gene, such as Fbx15 gene or Nanog gene, are selected in their living state among the differentiated cells into which the nuclear reprogramming factors have been introduced. The method for this selection is not particularly limited. For example, a marker gene, such as GFP gene or beta-galactosidase gene, may be knocked-in at downstream of a promoter for an endogenous Fbx15 gene or an endogenous Nanog gene, and cells expressing the marker gene may be selected. Alternatively, by knocking-in a drug-resistance gene, such as a neomycin resistance gene, a hygromycin tolerance gene, a puromycin resistance gene, etc., at downstream of a promoter for an endogenous Fbx15 gene or an endogenous Nanog gene, target cells can be easily selected on the basis of resistance to the drug.

Cells expressing the undifferentiation marker gene are thus selected among the differentiated cells into which the nuclear reprogramming factors have been introduced, and the resulting cell population is used as the induced pluripotent stem cells.

### == Methods for identifying and selecting secondary neurospheres having low risk of tumor formation after transplantation ==

When the induced pluripotent stem cells are administered to a vertebrate for a therapeutic purpose, it is preferred to make embryoid bodies (EBs) and allow the EBs to differentiate into neural stem cells in culture prior to the administration in order to enhance their ability to differentiate into neural cells. For example, the EBs may be formed in the presence of low concentration (10⁻⁹ M to 10⁻⁶ M) of retinoic acid. Alternatively, EBs may be formed by adding Noggin protein to culture medium of differentiated cell-derived pluripotent stem cells. Specifically, a conditioned medium in which Noggin has been transiently expressed by introducing Xenopus Noggin gene into cultured mammalian cells may be added to the culture medium (1 to 50% (v/v)). Recombinant Noggin protein (about 1 ug/ml) may also be used instead of the conditioned medium.

The EBs thus obtained are dissociated and cultured in a serum-free medium supplemented with FGF-2 (10 to 100 ng/ml), so that the cells differentiate into neural stem cells as neurospheres. The neurosphere directly derived from the EBs are referred to as primary neurosphere (also referred to as PS-PNS hereinafter), while the neurosphere obtained by dissociating the PS-PNS and allowing them to form neurospheres again under the same condition, as well as the neurosphere formed by repeating such neurosphere dissociation-neurosphere formation processes are collectively referred to as secondary neurosphere (also referred to as PS-SNS hereinafter).

The secondary neurospheres have different characteristics at each time when being differentiated from induced pluripotent stem cells. Each of the secondary neurospheres having different characteristics is herein referred to as a clone.

When vertebrate is transplanted with PS-SNS, tumor is sometimes formed in the transplantation site. This tumor formation attributed to the PS-SNS character. Some clones of PS-SNS have high risk of tumor formation, but some clones don't. The clones having low risk of tumor formation can be effectively identified and selected by examining promoter activity of Nanog gene as a marker among many clones of the PS-SNSs and selecting clones in which the promoter activity of Nanog gene is repressed. From this finding, present invention provides a method for identifying and selecting PS-SNSs having low risk of tumor formation after transplantation.

The method for examining the promoter activity of Nanog gene is not particularly limited; in the case where cells of the PS-SNS have a reporter gene whose expression is regulated by the promoter of Nanog gene, the expression of the reporter gene may be examined. Examples of the reporter gene include genes encoding fluorescent proteins such as GFP, YFP and BFP, luminescent proteins such as luciferase, and enzymes such as beta-galactosidase, alkaline phosphatase and HRP. Alternatively, the promoter activity of Nanog gene may be evaluated by examining expression of the endogenous Nanog gene.

Expression of a reporter gene or an endogenous gene may be evaluated by detecting transcription product (such as hnRNA or mRNA) by using PCR method, LAMP method or Northern hybridization method, as well as by detecting translation product (such as polypeptide or modified peptide) by using RIA method, IRMA method, EIA method, ELISA method, LPIA method, CLIA method, immunoblotting method or the like. Therefore, the product whose expression was driven by the promoter of Nanog gene, such as a transcription product or a translation product of a reporter gene or an endogenous gene, may be used as a marker for selecting PS-SNSs.

When examining the promoter activity of Nanog gene, an activation level in each cell in clones may be measured. While the method for measuring the activation level in each cell is not particularly limited, it is preferable to use the differentiated cell-derived pluripotent stem cells having a reporter gene and examine expression of the reporter under culturing conditions due to convenience of the detection. In this case, ratio of cells in which the promoter of Nanog gene is activated is calculated for each clone, and clones in which the promoter activity of Nanog gene is repressed, e.g. having the ratio of 0.01% or less, may be preferably selected and used. Alternatively, an activation level of the promoter of Nanog gene in a whole clone may be measured for each clone. While the method for measuring the activation level in a whole clone is not particularly limited, it is preferable to measure a transcription product of the reporter gene or the endogenous gene by using quantitative PCR due to convenience of the detection. In this case, it is preferable to examine the activation level of the promoter of Nanog gene and risk of tumor formation after transplantation in vivo and determine the threshold of the activation level at which tumor formation almost disappears in advance, so that the clones in which the promoter activity of Nanog gene is repressed, e.g. whose activation level is equal to or less than the threshold, should be selected.

The clones of PS-SNSs thus selected may be isolated to prepare pharmaceutical compositions or transplants for treatment of nervous system disease, especially nerve injury.

### == Pharmaceutical compositions ==

When using pharmaceutical composition or transplant containing the PS-SNSs, the methods which were developed for using ES cell-derived neural stem cells as a therapeutic agent for nerve injury can be employed (Okada et al. Dev. Biol. vol.275, pp.124-142, 2004).

The therapeutic agent for nerve injury may contain buffer containing salt etc., antibiotics, agent such as a preservative or the like, other than the PS-SNSs. The nervous tissue to be treated is not particularly limited, and it may be either in the central nervous system (the brain and the spinal cord) or the peripheral nervous system. Further, as long as the disease exhibits a pathological condition in which nerve cells are damaged, the disease to be treated and the cause of the disease are not specifically limited. The disease can be a traumatic disease such as a spinal cord injury; a neurodegenerative disease such as amyotrophic lateral sclerosis, Alzheimer's disease, Parkinson's disease, progressive supranuclear palsy, Huntington disease, multiple system atrophy, and spino-cerebellar degeneration; necrosis of nerve cells resulting from cerebral infarction or intracerebral hemorrhage. The cause of the disease can be the primary cause being associated with injury, cerebral infarction, etc.; the secondary cause being associated with infection, tumor, etc.

The PS-SNSs may be administered either directly or indirectly. For example, for a direct administration, they may be transplanted to the injured site of nerves; for an indirect administration, they may be injected intravenously or intraspinally and delivered to the affected site through the circulation of blood or cerebrospinal fluid.

### == Kits ==

Kits for selecting secondary neurospheres having low or no risk of tumor formation after transplantation, which are derived from differentiated cell-derived pluripotent stem cells, contain reagents for detecting the promoter activity of Nanog gene.

As mentioned above, the method for detecting the promoter activity of Nanog gene includes PCR, LAMP, Northern hybridization, RIA, IRMA, EIA, ELISA, LPIA, CLIA and immunoblotting, and since these methods have been well known, a person skilled in the art can include any appropriate solution, compound or the like in the kit in accordance with the detection method to be employed.

### Examples

### == Cells ==

In the following examples, the induced pluripotent stem cells were obtained by introducing a gene set of Oct3/4, Sox2, c-Myc and Klf4, or a gene set of Oct3/4, Sox2 and Klf4, as the nuclear reprogramming factors, to mouse embryonic fibroblasts. Specifically, in the case of the former gene set, Fbx15-iPS cells (Takahashi K, Yamanaka S. (2006). "Induction of pluripotent stem cells from mouse embryonic and adult fibroblast cultures by defined factors." Cell 126: 663-676) which are the cells selected by using the expression of Fbx15 gene as a marker or Nanog-iPS cells (Okita K, Ichisaka T, Yamanaka S. (2007). "Generation of germline-competent induced pluripotent stem cells.". Nature 448: 313-317) which are the cells selected by using the expression of Nanog gene as a marker were used. In the case of the latter gene set, Myc⁻-Nanog-iPS cells (Nakagawa M, Koyanagi M, Tanabe K, Takahashi K, Ichisaka T, Aoi T, Okita K, Mochiduki Y, Takizawa N, Yamanaka S. (2008). "Generation of induced pluripotent stem cells without Myc from mouse and human fibroblasts.". Nat Biotechnol 26: 101-106) which were selected by using the expression of Nanog gene as a marker were used.

### Example 1

### <Avoidance of tumor formation caused by iPS cell>

### == Preparation of iPS-SNS ==

The following clones were used as the Nanog-iPS cells in this example: the 20D17 Nanog-iPS clone and the as38C2 Nanog-iPS clone, to which T58A-c-Myc had been introduced, and the 38D2 Nanog-iPS clone, to which wild-type c-Myc had been introduced. As for the control, the Nanog-EGFP-ES clone of mouse ES cell was used (with regard to these clones, refer to Okita K, Ichisaka T, Yamanaka S. (2007). "Generation of germline-competent induced pluripotent stem cells.". Nature 448: 313-317).

To enhance the ability of these iPS cells to differentiate into neural cells, embryoid bodies (EBs) were formed in the presence of 10⁻⁸ M retinoic acid. The EBs were then cultured in a serum-free medium supplemented with 20 ng/ml FGF-2 (Okada et al. Dev. Biol. vol.275, pp. 124-142, 2004) and iPS-derived primary neurospheres (also referred to as iPS-PNS hereinafter) were formed after seven days. The iPS-PNSs were dissociated and cultured under the same conditions so that iPS-derived secondary neurospheres (also referred to as iPS-SNS hereinafter) were formed. The neurospheres were possible to be repeatedly formed by the same procedure. A similar treatment was applied to ES cells for the control experiment.

### == Identification and selection of a clone of iPS-SNS ==

First, the expression of Nanog gene in these iPS-SNS clones was examined. An exogenous EGFP gene whose expression is controlled by the Nanog promoter has been inserted to the genome of these cells in advance. The cells derived from each clone were dissociated and FACS analysis was conducted using EGFP as a marker to measure the ratio of cells in which the Nanog promoter was activated.

Then the cells of each clone were transplanted to the corpus striatum of NOD/SCID mice, and after four weeks or later, mice were dissected to examine the presence of tumor in the brain. As a result, the iPS-SNS clones in which about 0.01 % or more of cells had expressed EGFP formed tumor in the body where transplanted (TABLE 1). Especially, none of the clones derived from 38C2 showed the expression of EGFP, and there were mice who survived for 24 weeks without forming tumor (for example, mouse No.8 and mouse No.9 which had been transplanted with 38C2-N2 clone). There was no tumor formation when ES cells were transplanted. It should be noted that these tumors were confirmed to be teratomas by histological analyses.

**[Table 1]**

| Type of iPS | iPS clone | Clone | Ratio of GFP+(%) | Tumor formation | Number of mice analyzed | Date of analysis (day) |
|---|---|---|---|---|---|---|
| Nanog-iPS | 20D17 | N3 | 0.00363 | - | 4 | 6, 22, 22, 22 |
| | | N4 | 0.049 | + | 2 | 6, 22 |
| | | N5 | 0.11 | + | 4 | 4, 4, 6, 6 |
| | | N6 | 0.019 | + | 4 | 4, 4, 6, 6 |
| | 38D2 | N3 | 0.026 | + | 3 | 7, 22, 22 |
| | | N4 | 0.00143 | - | 2 | 6, 22 |
| | | N5 | 0.00779 | - | 2 | 22, 22 |
| | | N6 | 0.38 | + | 4 | 6, 6, 6, 8 |
| | 38C2 | N3 | 0 | - | 3 | 4, 4, 4, |
| | | N4 | 0 | - | 2 | 4, 4 |
| Myc⁻-Nanog-iPS | Ng178B5 | N2 | 0 | - | 4 | 4, 4, 4, live |
| Nanog-EGFP-ES | 1A2 | N2 | 0 | - | 4 | 4, 4, live, live |
| | | N3 | 0 | - | 4 | 4, 4, 4, 4, |

| | | | | | | |
|---|---|---|---|---|---|---|
| -: tumor formation absent; +: tumor formation present live : surviving without forming tumor | | | | | | |

As shown in TABLE 1, the iPS-SNS clones in which less than about 0.01% of cells had expressed EGFP had lowered tumor formation capability in the body when transplanted.

### Example 2

### <Treatment of spinal cord-injured mice using iPS-SNSs>

### == iPS cells used and preparation of iPS-SNS cells ==

In this Example, iPS-SNSs were prepared similarly as in Example 1 by using 38C2-Nanog-iPS-SNS and Ng178B5-Myc⁻-Nanog-iPS-SNS, both of which had been confirmed to show no tumor formation capability in Example 1. As for a control, a similar experiment was performed using EB3-ES-SNS.

In addition, to obtain clones expressing a red light-emitting click beetle luciferase (CBRluc) and a red fluorescent protein (RFP) as the markers for transplanted 38C2-Nanog-iPS-SNS cells, a lentivirus vector harboring an IRES flanked by CBRluc gene and REP gene was introduced into the cells. The resulting CBRluc-38C2-Nanog-iPS was used for a transplantation experiment into spinal cord-injured mice.

### == Generation of spinal cord-injured mice and cell transplantation ==

Model mice of spinal cord injury were made by inducing traumatic spinal cord injury of the spinal nerve at the 10th thoracic vertebral level, and transplantation of 38C2-Nanog-iPS-SNS or Ng178B5-Myc--Nanog-iPS-SNS was performed as follows.

First, 8- to 9-week-old C57B16 female mice weighing 20 to 22 g were anesthetized with ketamine (100 mg/kg) and xylazine (10 mg/kg). After laminectomy of the 10th thoracic vertebra, the dorsal surface of the dura mater was exposed, and traumatic spinal cord injury was produced using the Infinite Horizon Impactor (60 kdyn; Precision Systems, Kentucky, IL).

To transplant cells to the injured spinal cord, the injury site was exposed again at nine days after the injury. Cells of 5 x 10⁵ cells/2ul which were obtained by dissociating the secondary neurospheres were introduced into the center of the defective area at a rate of 0.5 ul/min using a glass micropipette mounted on a stereotaxic injector (KDS310, Muromachi-kikai, Tokyo, Japan). As a control, culture media without cells were injected in the same method as the cell transplantation.

### == Analysis of spinal cord-injured mice transplanted with 38C2-Nanog-iPS-SNS ==

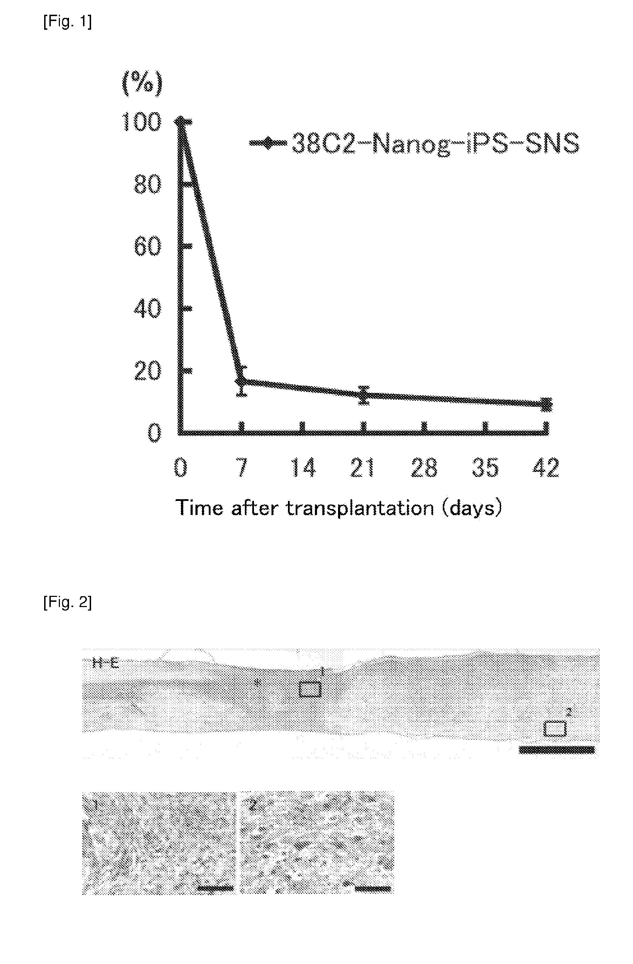

In a bioluminescent imaging (BLI) analysis (Okada et al., Faseb J. vol.19, pp.1839-1841, 2005), intensities of luminescence by luciferase were measured immediately after transplantation, at days 7, 21, and 35, and were used as indexes of the number of cells. Specifically, D-luciferin (150 mg/kg body weight) was intraperitoneally injected into the mice. For 15 to 40 min after administration of the luciferin, images of the mice with the field-of-view set at 10 cm were repeatedly taken until the highest intensity was obtained. All the images were analyzed using Igor software (WaveMetrics, Lake Oswego, OR) and Living Image software (Xenogen, Alameda, CA). To quantify the number of photons, a fixed transplantation area was defined and analyzed in each of the mice. Using the number of photons obtained at each time point, ratios to the initial value were calculated and plotted in FIG. 1. As shown in FIG. 1, approximately 60% of the transplanted cells were lost by day 7 after the transplantation, and then the signals of the transplanted cells were gradually reduced. At day 35, about 18% of the transplanted cells were found to be alive.

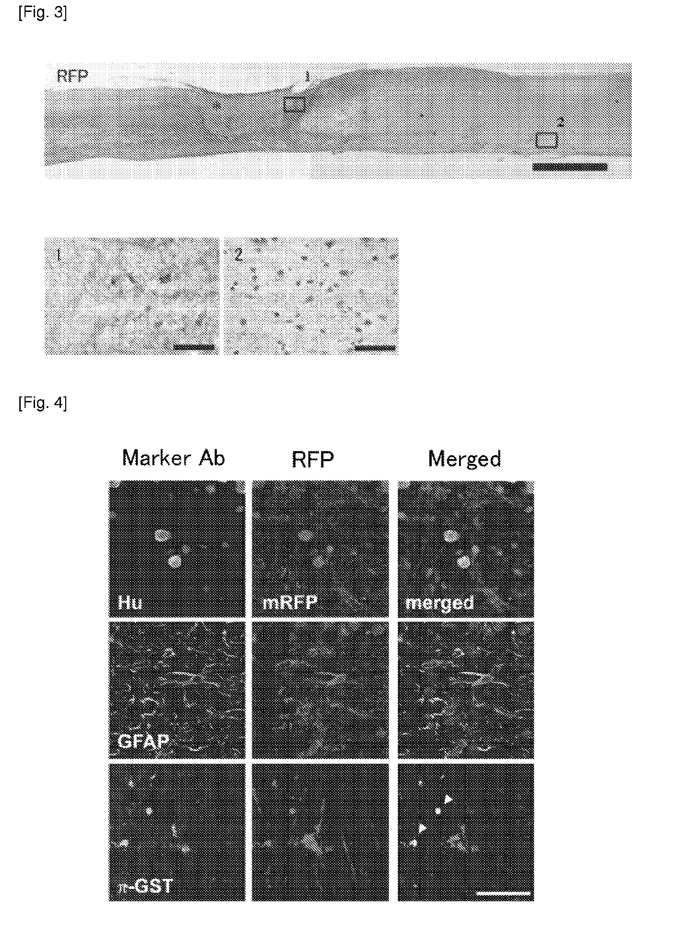

At six weeks after the traumatic injury, histological analyses of the 38C2-Nanog-iPS-SNS cell-transplanted mice were performed. In the panel H-E of FIG. 2 (hematoxylin and eosin staining) and in the panel RFP of FIG. 3 (DAB staining using HRP-conjugated anti-RFP antibody), an asterisk indicates the cell transplantation site, and magnified images of the areas indicated by square 1 (the periphery of the nerve injury area) and square 2 (the white matter in the anterior of the transplantation site), respectively, are shown at the bottom. As a result, no sign of tumorigenesis was observed (FIG. 2). It should be noted that while most of the transplanted cells were observed around the nerve injury area (FIG. 3-1), there were also some cells that had moved toward the anterior by approximately 4 mm (FIG. 3-2).

Sections of the injured spinal cord were stained using antibodies against cell-type specific markers. The 38C2-derived transplanted cells detected with RFP were found to have differentiated into three cell species: neuronal cells (FIG. 4A; the marker was Hu), astrocytes (FIG. 4B; the marker was GFAP), and oligodendrocytes (FIG. 4C; the marker was GST-pi) (FIG. 4).

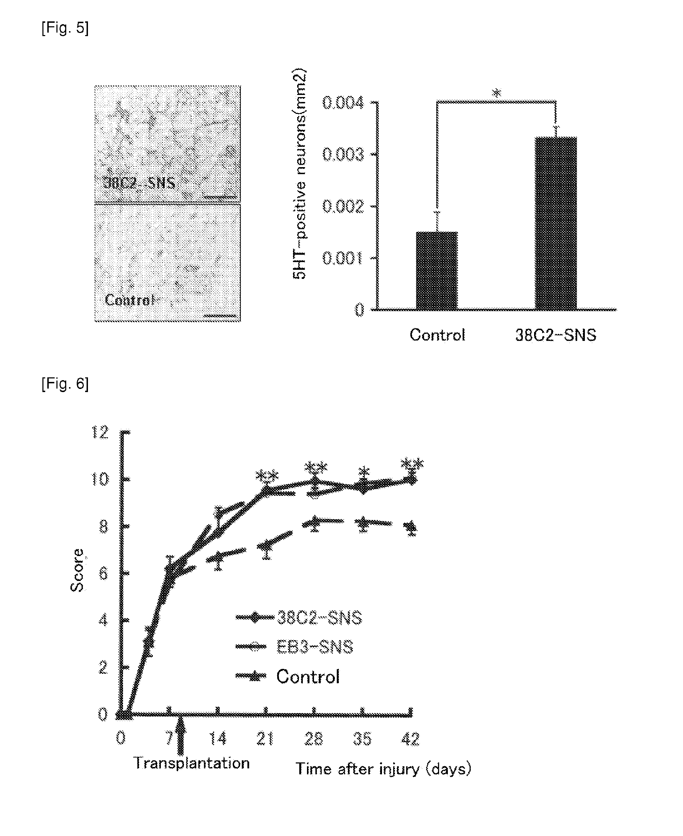

Further, to examine the number of serotonergic neurons, staining was performed using an anti-serotonin receptor antibody. The images obtained by microscopic observation as well as the measured areas of the stained portions to quantify the numbers of neurons are shown in FIG. 5. There were significant reduction of the number of the 5HT-positive serotonergic neurons in both the microscope images and the stained areas when the culture medium without cells (which is indicated by "Control" in FIG.5) was injected as compared with the number of the neurons when 38C2-iPS-SNS (which is indicated by "38C2-SNS" in FIG.5) was transplanted (FIG. 5).

Next, the motor function of hindlimbs was evaluated by the Basso-Beattie-Bresnahan (BBB) score (Basso et al., J. Neurotrauma vol.12, pp.1-21, 1995) at every seven days till day 42. In the motor function analysis, comparison was made among the three groups: 38C2-Nanog-iPS-SNS-transplanted group (n=20), EB3-ES-SNS-transplanted group (n=15), and the group receiving only the cell-free medium (n=12). In all the three groups, mice were completely paralyzed immediately after the induction of the spinal cord injury, but all gradually recovered. However, at six weeks after the operation, the mice in the medium-injected group were unable to support their weight on the hindlimbs, whereas the mice in the 38C2-Nanog-iPS-SNS-transplanted group recovered enough to be able to lift their trunks. As the result of the comparison of the BBB scores, almost the same degree of recovery was observed in the 38C2-Nanog-iPS-SNS-transplanted group (10.03+/-0.47) and the EB3-ES-SNS-transplanted group (10.10+/-0.24) at the sixth postoperative week, with a significant difference from the recovery in the group receiving only the cell-free medium (8.08+/-0.39) (FIG. 6). Also in clinical observations, the 38C2-Nanog-iPS-SNS-transplanted mice exhibited marked recovery of weight-supported plantar stepping. It should be noted that in the case where Ng178B5-Myc⁻ - Nanog-iPS-SNS was used, therapeutic effect was observed at a similar level to the case where 38C2-Nanog-iPS-SNS was used.

In conclusion, by transplanting iPS cells-derived SNS cells to nerve injured mice, the nerve injury can be treated.

### Industrial Applicability

In accordance with the present invention, methods for efficiently identifying and selecting a secondary neurosphere having low or no risk of side effects derived from an induced pluripotent stem cell, as well as a secondary neurosphere selected by the methods and use of the secondary neurosphere, can be provided.

## Claims

1. A method for selecting a clone of secondary neurospheres having low or no risk of tumor formation after transplantation among clones of secondary neurospheres derived from an induced pluripotent stem cell, comprising the steps of:
examining promoter activity of Nanog gene in each of the clones; and
selecting a clone in which the promoter activity of Nanog gene is repressed.

2. A method for preparing a clone of secondary neurospheres comprising preparing clones of secondary neurospheres from induced pluripotent stem cells, and selecting a clone of secondary neurospheres according to the method of claim 1.

3. The method according to Claim 1 or 2, wherein the secondary neurospheres comprise a marker gene whose expression is regulated by the promoter of Nanog gene, and expression of the marker gene is examined.

4. The method according to Claim 3, wherein the marker gene encodes a fluorescent protein, a luminescent protein, or an enzyme.

5. The method according to Claim 1 or 2, wherein expression of the endogenous Nanog gene is examined.

6. The method according to any one of Claims 1 to 5, wherein an activation level of the promoter of Nanog gene is measured in each cell belonging to each of the secondary neurospheres.

7. The method according to Claim 6, wherein ratio of cells in which the promoter of Nanog gene is activated is calculated for each of the clones.

8. The method according to Claim 7, wherein a neurosphere whose ratio of the cells in which the promoter of Nanog gene is activated is 0.01% or less is selected.

9. The method according to any one of Claims 1 to 5, wherein an activation level of the promoter of Nanog gene is measured as a whole in each of the secondary neurospheres.

## Patentansprüche

1. Verfahren zum Auswählen eines Klons von sekundären Neurosphären, die nach einer Transplantation nur ein geringes oder gar kein Risiko einer Tumorbildung aufweisen, aus Klonen von sekundären Neurosphären, die von einer induzierten pluripotenten Stammzelle abgeleitet sind, umfassend die Schritte:
Untersuchen der Promotoraktivität des Nanog-Gens bei jedem der Klone; und
Auswählen eines Klons, bei dem die Promotoraktivität des Nanog-Gens unterdrückt ist.

2. Verfahren zur Herstellung eines Klons von sekundären Neurosphären, umfassend das Herstellen von Klonen von sekundären Neurosphären aus induzierten pluripotenten Stammzellen und Auswählen eines Klons von sekundären Neurosphären nach dem Verfahren gemäß Anspruch 1.

3. Verfahren gemäß Anspruch 1 oder 2, wobei die sekundären Neurosphären ein Marker-Gen, dessen Expression durch den Promotor des Nanog-Gens reguliert wird, umfassen und die Expression des Marker-Gens untersucht wird.

4. Verfahren gemäß Anspruch 3, wobei das Marker-Gen ein fluoreszierendes Protein, ein lumineszierendes Protein oder ein Enzym codiert.

5. Verfahren gemäß Anspruch 1 oder 2, wobei die Expression des endogenen Nanog-Gens untersucht wird.

6. Verfahren gemäß einem der Ansprüche 1 bis 5, wobei in jeder Zelle, die zu jedem der sekundären Neurosphären gehört, das Aktivierungsniveau des Promotors des Nanog-Gens gemessen wird.

7. Verfahren gemäß Anspruch 6, wobei der Anteil von Zellen, bei denen der Promotor des Nanog-Gens aktiviert wird, für jeden der Klone berechnet wird.

8. Verfahren gemäß Anspruch 7, wobei eine Neurosphäre, bei der der Anteil der Zellen, bei denen der Promotor des Nanog-Gens aktiviert ist, 0,01% oder weniger beträgt, ausgewählt wird.

9. Verfahren gemäß einem der Ansprüche 1 bis 5, wobei in jeder der sekundären Neurosphären das Aktivierungsniveau des Promotors des Nanog-Gens als Ganzes gemessen wird.

## Revendications

1. Méthode de sélection d'un clone de neurosphères secondaires, dont le risque de former une tumeur après transplantation est faible ou inexistant, parmi des clones de neurosphères secondaires dérivant d'une cellule souche pluripotente induite, comprenant les étapes suivantes :
l'examen de l'activité de promoteur du gène Nanog dans chacun des clones ; et la sélection d'un clone dans lequel l'activité de promoteur du gène Nanog est réprimée.

2. Méthode de préparation d'un clone de neurosphères secondaires, comprenant la préparation de clones de neurosphères secondaires à partir de cellules souches pluripotentes induites, et la sélection d'un clone de neurosphères secondaires selon la méthode de la revendication 1.

3. Méthode selon la revendication 1 ou 2, dans laquelle les neurosphères secondaires comprennent un gène marqueur dont l'expression est régulée par le promoteur du gène Nanog, et l'expression du gène marqueur est examinée.

4. Méthode selon la revendication 3, dans laquelle le gène marqueur code une protéine fluorescente, une protéine luminescente ou une enzyme.

5. Méthode selon la revendication 1 ou 2, dans laquelle l'expression du gène Nanog endogène est examinée.

6. Méthode selon l'une quelconque des revendications 1 à 5, dans laquelle un niveau d'activation du promoteur du gène Nanog est mesuré dans chaque cellule appartenant à chacune des neurosphères secondaires.

7. Méthode selon la revendication 6, dans laquelle la proportion de cellules dans lesquelles le promoteur du gène Nanog est activé est calculée pour chacun des clones.

8. Méthode selon la revendication 7, dans laquelle une neurosphère dont la proportion de cellules dans lesquelles le promoteur du gène Nanog est activé est inférieure ou égale à 0,01 % est sélectionnée.

9. Méthode selon l'une quelconque des revendications 1 à 5, dans laquelle un niveau d'activation du promoteur du gène Nanog est mesuré dans son ensemble dans chacune des neurosphères secondaires.
